Europäisches Patentamt

**European Patent Office**

Office européen des brevets

(19)

(11) Publication number: **0 010 902**
**B1**

(12) **EUROPEAN PATENT SPECIFICATION**

(45) Date of publication of patent specification: **31.03.82**

(21) Application number: **79302245.0**

(22) Date of filing: **17.10.79**

(51) Int. Cl.³: **C 07 C 57/04,**
**C 07 C 51/215,**
**C 07 C 57/05,**
**B 01 J 23/16, B 01 J 27/18**

(54) Process for the production of (meth)acrylic acid by the catalytic vapour phase oxidation of isobutane or propane.

(30) Priority: **30.10.78 US 956011**

(43) Date of publication of application:
**14.05.80 Bulletin 80/10**

(45) Publication of the grant of the patent:
**31.03.82 Bulletin 82/13**

(84) Designated Contracting States:
**BE CH DE FR GB IT**

(56) References cited:
**GB - A - 1 340 891**
**US - A - 3 470 239**
**US - A - 3 956 378**

(73) Proprietor: **Rohm and Haas Company**
**Independence Mall West**
**Philadelphia, Pennsylvania 19105 (US)**

(72) Inventor: **Krieger, Harold**
**61 West School House Lane**
**Philadelphia Pennsylvania 19144 (US)**
Inventor: **Kirch, Lawrence Samuel**
**8710 Oriole Lane**
**Huntingdon Valley Pennsylvania 19006 (US)**

(74) Representative: **Wood, Peter Worsley Spencer**
**et al,**
**Rohm and Haas Company Patent Department**
**Chesterfield House Barter Street**
**London, WC1A 2TP (GB)**

Courier Press, Leamington Spa, England.

Process for the production of (meth)acrylic acid by the catalytic vapour phase oxidation of isobutane or propane

This invention concerns the production of (meth)acrylic acid by the catalytic vapour phase oxidation of isobutane or propane respectively.

It is known (*vide* U.S. Patent No. 3,293,290) to prepare acrylic acid by the vapour phase catalytic oxidation of propane using as catalyst phosphomolybdic acid or its iron and bismuth salts. However conversions and selectivities with the known processes are in need of improvement.

It is also known (*vide* U.K. Patent Specification No. 1,340,891) that methacrylic acid may be prepared from isobutane by direct vapour phase catalytic oxidation using catalysts containing molybdenum, antimony and oxygen. However, such process suffers from the disadvantage of a very low selectivity when converting the isobutane to methacrylic acid. The selectivity is known to be of the order of about 2% and this makes the process commercially unattractive.

The present invention provides a process for the preparation of acrylic or methacrylic acid which comprises reacting, at an elevated temperature and in the vapour phase, oxygen and propane or isobutane respectively over a catalyst which in the case of propane contains molybdenum, phosphorus and oxygen and in the case of isobutane contains antimony, molybdenum and oxygen, characterised in that (a) there is used for the oxidation of the propane or isobutane a catalyst which contains $Mo_x, Sb_y, P_z$ and combined oxygen wherein x, y and z represent the atomic ratios of Mo, Sb and P and x is 9 to 24, y is 0.25 to 2 and z is 0.5, particularly 0.75, to 2, (b) the amount of propane or isobutane in the feed gas is from 10 to 70 mole per cent and (c) the propane or isobutane is contacted with the catalyst at a temperature from 200°C to 450°C.

The use of the specific catalyst employed according to the invention, in conjunction with the other process conditions, results in a significantly higher selectivity in the conversion of isobutane or propane to (meth)acrylic acid.

The molar ratio of oxygen to propane or isobutane in the feed gas is preferably between about .05:1 to about 1:1 and more preferably between 0.1:1 to about 0.4:1. Any source of oxygen may be used. From an economic point of view, it is preferred that the source of oxygen be air.

If the molar ratio of oxygen to alkane is less than about .05:1, then the economics of the process will be adversely affected in that the amount of oxygen present will be insufficient for the optimum conversion of the alkane to methacrylic or acrylic acid. If however, the molar ratio of oxygen to alkane exceeds about 1:1 then there is the possibility that the oxidation will proceed too far and excessive amounts of carbon dioxide will be formed.

Hence the preference for a molar ratio of oxygen to alkane of from about 0.05:1 to about 1:1.

Diluent gases are usually present in the alkane containing feed gas. Although any diluent gas may be used which does not adversely affect the oxidation, it is most convenient to use, as a diluent gas, the nitrogen which is present in air which also serves as the source of supply for the oxygen which is needed for the oxidation of the alkane to methacrylic or acrylic acid.

As regards the catalyst, it has been found that using a catalyst in accordance with the invention to oxidize isobutane, it is possible to achieve selectivities as high as 50 per cent. If the atomic ratios of the elements Mo,Sb and P lie outside the ranges indicated above the efficacy of the oxidation of propane or isobutane to the desired product will be adversely affected.

The atomic ratios of the various elements of the catalyst to one another are preferably from about 12 to about 15 of molybdenum; about 1 to about 2 of antimony; and about 1 to about 1.25 of phosphorous, more preferably an atomic ratio Mo:Sb:P of 12:1:1.

The catalyst may be, and preferably is, prepared by adding antimony pentachloride to a solution containing nitric acid and ammonium hydroxide in deionized water, phosphomolybdic acid then being added to the resultant slurry. The slurry is then stirred with heating for about 16 hours at a temperature of about 50°C. It is thereafter evaporated and dried under vacuum. The solid thereby obtained is then calcined for from about 6 to about 24 hours at a temperature of from about 375°C to about 425°C and preferably from about 395°C to about 405°C. The calcined product is then crushed and particles in the 8 to 30 mesh (U.S. Standard Sieve Series) range are then used.

When water is present in the feed, the selectivity of the alkane to acrylic or methacrylic acid is improved. Generally, the molar ratio of water to alkane should be from about 1:5 to about 5:1. Bacause water is formed in the course of the reaction, it may not be necessary to add water vapour to the feed gas. It is preferred however that the molar ratio of water to alkane should be from about 1:3 to about 3:1 as excellent results have thereby been obtained.

The pressure at which the reaction is conducted may vary widely and may, for example, be from atmospheric to about 5 atmospheres. It is preferred however that the reaction be conducted at a pressure of from about 1 to about 2 atmospheres because the results at such pressure range have been optimal.

The contact time of the feed gas with the catalyst will vary depending upon the desired conversion and the partial pressure of the alkane in the feed gas. Generally, the contact time will be between about 1 and 20 seconds. The contact time may readily be determined by dividing the bulk volume of the catalyst by the volumetric gas flow rate measured at standard temperature and pressure.

The process may be conducted by passing the feed gas over the catalyst for the desired contact time and at the indicated temperature. It has been found advantageous to pre-heat the feed gas prior to contacting it with the catalyst.

The products of the catalyst oxidation of the alkane are acrylic acid or methacrylic acid, methacrolein or acrolein, carbon monoxide, carbon dioxide, acetic acid and acetone.

The methacrylic or acrylic acid can be separated from the other oxidation products and purified in any suitable manner. Purification of methacrylic or acrylic acid is well known. The methacrolein or acrolein and any unreacted alkane can be recycled by addition to the feed gas which is to be passed in contact with the catalyst. If desired, the acetic acid may be recovered as a by-product.

In order to more fully illustrate the nature of this invention and the manner of practising the same, the following examples are presented.

### Example 1—(A) Catalyst Preparation

16.4 gms of antimony pentachloride is added to a solution containing 19.6 gms to 70 per cent nitric acid and 26.5 gms of 30 per cent ammonium hydroxide in 800 gms of deionized water. 130 gms of phosphomolybdic acid is then added to the resultant slurry. The slurry is then stirred with heating for 16 hours, whereupon the water is evaporated and the mixture is dried under vacuum. The solid which is thereby obtained is calcined for 6 hours at a temperature of 400°C. The calcined product is then crushed and the particles in the 8 to 30 mesh (U.S. Standard Sieve Series) range are used.

### (B) Methacrylic Acid Preparation

25 g of the catalyst prepared above is charged to a fixed bed tubular reactor having 3/8 inch diameter stainless steel tubing. The reactor is then immersed in a molten salt bath to provide heat transfer.

A gaseous mixture containing (mol. per cent) 10 per cent isobutane, 12 per cent oxygen, 48 per cent nitrogen and 30 per cent steam is passed over the catalyst. The temperature at which the feed gas is passed over the catalyst is 340°C. The reactor pressure is maintained at 1,36 atmospheres (20 psig). The contact time of feed gas with catalyst is 6.1 seconds.

10 per cent of the isobutane which is fed to the reactor is converted to oxidized products with a 50 per cent selectivity to methacrylic acid and a 20 per cent selectivity to methacrolein, which is recycled.

### Example 2—Acrylic Acid Preparation

The catalyst of Example 1A and the equipment of Example 1B are used. A gaseous mixture containing (mol. per cent) 15% propane, 10% oxygen, 40% nitrogen and 35% steam is passed over 25 grams of the catalyst. The contact time of the feed gas with the catalyst is 5.5 seconds. The salt bath temperature is 340°C and the reactor pressure is 1,36 atmospheres (20 psig).

Ten per cent of the propane is converted to oxidized products with a 19% selectivity to acrylic acid.

### Example 3—Methacrylic Acid Preparation

25 g of the catalyst of Example 1A is packed into a 3/8 inch diameter tubular reactor of Example 1B. The procedure of Example 1B is repeated except that the composition of the feed gas is (mol. per cent) 28.4% isobutane, 28.7% molecular oxygen and 42.9% steam. The contact time of feed gas with catalyst is 4.1 seconds. 9% of the isobutane is converted to oxidized products with a 46% selectivity to methacrylic acid and a 10% selectivity to methacrolein.

### Claims

1. A process for the preparation of acrylic or methacrylic acid which comprises reacting, at an elevated temperature and in the vapour phase, oxygen and propane or isobutane respectively over a catalyst which in the case of propane contains molybdenum, phosphorus and oxygen and in the case of isobutane contains antimony, molybdenum and oxygen, characterised in that (a) there is used for the oxidation of the propane or isobutane a catalyst which contains $Mo_xSb_yP_z$ and combined oxygen wherein x, y and z represent the atomic ratios of Mo, Sb and P and x is 9 to 24, y is 0.25 to 2 and z is 0.5 to 2, (b) the amount of propane or isobutane in the feed gas is from 10 to 70 mole per cent and (c) the propane or isobutane is contacted with the catalyst at a temperature from 200°C to 450°C.

2. A process according to Claim 1, wherein the atomic ratio of molybdenum to antimony to phosphorous is 12 to 1 to 1 respectively.

3. A process according to Claim 1 or 2, wherein the feed gas contains oxygen and propane or isobutane in a molar ratio from 0.05:1 to 1:1.

4. A process according to Claim 3, wherein the molar ratio of oxygen to propane or isobutane is from 0.1:1 to 0.4:1.

5. A process according to any preceding claim, wherein the water vapour is present in the feed gas.

6. A process according to Claim 5, wherein the water vapour is present in an amount so that the molar ratio of water to alkane is from 1:5 to 5:1.

7. A process according to any preceding claim, wherein the feed gas is contacted with the catalyst at a pressure of from 1 atmosphere to 5 atmospheres.

8. A process according to any preceding claim, wherein the contact time of the feed gas with the catalyst is from 1 to 20 seconds.

9. A process according to any preceding claim, wherein said feed gas contains propane or isobutane in an amount of from 20 to 50 mol. per cent.

## Revendications

1. Procédé pour la préparation d'acide acrylique ou d'acide méthacrylique qui comprend la réaction, à une température élevée et en phase gazeuse, d'oxygène et respectivement de propane ou d'isobutane sur un catalyseur qui dans le cas du propane contient du molybdène, du phosphore et de l'oxygène, et dans le cas de l'isobutane contient de l'antimoine, du molybdène et de l'oxygène, caractérisé en ce que (a) on utilise pour l'oxydation du propane ou de l'isobutane un catalyseur qui contient $Mo_x$, $Sb_y$, $P_z$ et de l'oxygène combiné où x, y et z représentent les rapports atomiques de Mo, Sb et P et x a une valeur de 9 à 24, y a une valeur de 0,25 à 2 et z a une valeur de 0,5 à 2, (b) la quantité de propane ou d'isobutane dans le gaz d'alimentation est de 10 à 70 moles % et (c) le propane ou l'isobutane est mis en contact avec le catalyseur à une température de 200 à 450°C.

2. Procédé selon la revendication 1 dans lequel le rapport atomique du molybdène/antimoine/phosphore est de 12/1/1.

3. Procédé selon l'une des revendications 1 ou 2 dans lequel le gaz d'alimentation contient de l'oxygène et du propane ou de l'isobutane dans un rapport molaire de 0,5/1 à 1/1.

4. Procédé selon la revendication 3 dans lequel le rapport molaire de l'oxygène au propane ou à l'isobutane est de 0,1/1 à 0,4/1.

5. Procédé selon l'une quelconque des revendications précédentes dans lequel de la vapeur d'eau est présente dans le gaz d'alimentation.

6. Procédé selon la revendication 5 dans lequel la vapeur d'eau est présente en une quantité telle que le rapport molaire de l'eau à l'alcane soit de 1/5 à 5/1.

7. Procédé selon l'une quelconque des revendications précédentes dans lequel on met le gaz d'alimentation en contact avec le catalyseur sous une pression de 1 à 5 x $10^5$ Pa.

8. Procédé selon l'une quelconque des revendications précédentes dans lequel la durée de contact du gaz d'alimentation avec le catalyseur est de 1 à 20 secondes.

9. Procédé selon l'une quelconque des revendications précédentes dans lequel le gaz d'alimentation contient du propane ou de l'isobutane à raison de 20 à 50 moles %.

## Patentansprüche

1. Verfahren zur Herstellung von Acrylsäure oder Methacrylsäure durch Umsetzen von Sauerstoff oder Propan bzw. Isopropan bei erhöhter Temperatur in der Dampfphase über einem Katalysator, der im Falle von Propan Molybdän, Phosphor und Sauerstoff und im Falle von Isobutan Antimon, Molybdän und Sauerstoff enthält, dadurch gekennzeichnet, daß a) für die Oxidation von Propan oder Isobutan ein Katalysator verwendet wird, der $Mo_x$, $Sb_y$, $P_z$ und gebundenen Sauerstoff enthält, wobei x, y und z für die Atomverhältnisse von Mo, Sb und P steht, und x 9 bis 24 ist, y 0,25 bis 2 ist und z 0,05 bis 2 ist, b) die Menge an Propan oder Isobutan in dem Beschickungsgas zwischen 10 und 70 Mol% schwankt und c) das Propan oder Isobutan mit dem Katalysator bei einer Temperatur von 200°C bis 450°C kontaktiert wird.

2. Verfahren nach Anspruch 1, dadurch gekennzeichnet, daß ein Atomverhältnis Molybdän:Antimon:Phosphor von 12:1:1 eingehalten wird.

3. Verfahren nach Anspruch 1 oder 2, dadurch gekennzeichnet, daß das eingesetzte Beschickungsgas Sauerstoff und Propan oder Isobutan in einem Molverhältnis von 0,05:1 bis 1:1 enthält.

4. Verfahren nach Anspruch 3, dadurch gekennzeichnet, daß ein Molverhältnis Sauerstoff:Propan oder Isobutan von 0,1:0,4:1 eingehalten wird.

5. Verfahren nach einem der vorhergehenden Ansprüche, da durch gekennzeichnet, daß Wasserdampf in dem eingesetzten Beschickungsgas vorliegt.

6. Verfahren nach Anspruch 5, dadurch gekennzeichnet, daß der Wasserdampf in einer solchen Menge vorliegt, daß das Molverhältnis von Wasser zu Alkan 1:5 bis 5:1 beträgt.

7. Verfahren nach einem der vorhergehenden Ansprüche, dadurch gekennzeichnet, daß das Beschickungsgas mit dem Katalysator unter einem Druck von 1 Atmosphäre bis 5 Atmosphären kontaktiert wird.

8. Verfahren nach einem der vorhergehenden Ansprüche, dadurch gekennzeichnet, daß die Kontaktzeit des Beschikkungsgases mit dem Katalysator zwischen 1 und 20 Sekunden liegt.

9. Verfahren nach einem der vorhergehenden Ansprüche, dadurch gekennzeichnet, daß das eingesetzte Beschickungsgas Propan oder Isobutan in einer Menge von 20 bis 50 Mol% enthält.